Europäisches Patentamt

European Patent Office

Office européen des brevets

(11) Veröffentlichungsnummer: **0 276 783**
**A2**

# (12) EUROPÄISCHE PATENTANMELDUNG

(21) Anmeldenummer: **88100975.7**

(51) Int. Cl.4: **A61K 39/002** , **A61K 39/008**

(22) Anmeldetag: **22.01.88**

Der (Die) Mikroorganismus (Mikroorganismen) ist (sind) bei der Deutsche Sammlung von Mikroorganismen unter der (den) Nummer(n) 4308 hinterlegt worden.

(30) Priorität: **29.01.87 DE 3702641**

(43) Veröffentlichungstag der Anmeldung:
**03.08.88 Patentblatt 88/31**

(84) Benannte Vertragsstaaten:
**AT BE CH DE FR GB IT LI NL SE**

(71) Anmelder: **Max-Planck-Gesellschaft zur Förderung der Wissenschaften e.V.
Bunsenstrasse 10
D-3400 Göttingen(DE)**

(72) Erfinder: **Overath, Peter, Prof. Dr.
Lessingweg 11
D-7400 Tübingen(DE)**
Erfinder: **Russel, David, Dr.
86-20 57th Road
Elmhurst New York 11373(US)**
Erfinder: **Alexander, James, Dr.
4 Prince Albert Road
Glasgow G12 9JX Scotland(GB)**

(74) Vertreter: **Huber, Bernhard, Dipl.-Chem. et al
Möhlstrasse 22 Postfach 860 820
D-8000 München 86(DE)**

(54) **Parasitose-Impfstoff.**

(57) Es wird ein Impfstoff gegen Parasitosen wie Leishmaniasis beschrieben, der als Wirkstoff wenigstens ein Membranantigen des Parasiten in die Lipiddoppelschicht von Liposomen inkorporiert enthält, das einen Schutz gegen Infektion mit dem homologen Parasiten hervorruft. Als Membran-Antigen wird z. B. das Glykoprotein gp63 oder das Glykolipid EF verwendet.

## FIG.1

EP 0 276 783 A2

## Parasitose-Impfstoff

Die Erfindung betrifft einen Parasitose-Impfstoff.

Die Leishmaniasis ist eine Tropenkrankheit, die sich nicht nur in den Tropen in immer größerem Ausmaß verbreitet, sondern auch in gemäßigten Zonen auftritt. Nach Schätzungen der Weltgesundheitsbehörde leiden zur Zeit ca. 50 bis 75 Millionen Menschen an dieser Krankheit.

Die menschliche Leishmaniasis tritt in drei Krankheitsformen auf, nämlich der kutanen, der mukokutanen und der viszeralen Form. Das klinische Bild hängt sowohl von der Art des Erregers als auch von der Immunantwort des befallenen Menschen ab; d. h. der gleiche Leishmanien-Stamm kann in verschiedenen Menschen zu verschiedenen Krankheitsbildern führen. So sind z. B. Leishmania mexicana und L. major Verursacher der kutanen Leishmaniasis in der neuen und der alten Welt. Obgleich im allgemeinen selbstheilend, wird die von diesen Parasiten verursachte Krankheit häufig von Komplikationen und hoher Morbidität begleitet (vgl. z. B. Biologie in unserer Zeit, 14, (1984) 111).

Zur Zeit stehen keine zufriedenstellenden Impfstoffe oder andere Mittel und Methoden zur Behandlung der Leishmaniasis (Leishmania spp) zur Verfügung. Die als Anti-Leishmania-Mittel verwendeten Antibiotika, wie z. B. Rifampicin, Antimonpräparate und Diamidine verursachen zum Teil verheerende Spätschäden, wie z. B. Diabetes oder Polyneuritis und führen manchmal sogar zum Tod.

Jüngste Untersuchungen, bei denen Menschen mit lebenden Promastigoten oder rohen Parasiten-Extrakten geimpft wurden, haben zwar gezeigt, daß es prinzipiell möglich sein sollte, einen Impfstoff gegen Leishmaniasis zu erhalten (C.H. Greenblatt in New Developments with Human and Veterinary Vaccines, Alan R. Liss Inc. New York, 1980, 259-291; W. Mayrink et al., Ann. Trop. Med. Parasitol 79 259-269 (1985)). Experimente an Mäusen haben jedoch ergeben, daß bestimmte Impfungsarten mit möglichen Schutz-Antigenen zu einer Verschlimmerung nachfolgender Infektionen führen können (F.Y. Liew et al, J. Immunol. 135 (1985) 2095-2101; F.Y. Liew et al, J. Immunol. 135 (1985) 2102-2107; R.G. Titus et al, J. Immunol. 135 (1985) 2108). Zur Schaffung eines erfolgreichen Impfstoffes ist es deshalb nicht nur erforderlich, ein geeignetes Schutz-Antigen zu identifizieren, sondern es muß auch in einer geeigneten Darreichungsform und Darreichungsart vorliegen und verwendet werden.

Ähnliche Verhältnisse liegen bei anderen Parasiten vor, insbesondere solchen, die durch Protozoen verursacht werden wie z. B. Amöbenruhr (Amöbiasis), Lambliose, Malaria, Trypasomoniasis, Toxoplasmose und Babesiose. In allen Fällen stehen entweder überhaupt keine Impfstoffe oder nur Impfstoffe mit nicht befriedigenden Eigenschaften zur Verfügung.

Aufgabe der vorliegenden Erfindung ist es deshalb, einen Impfstoff gegen Parasitosen, wie insbesondere Leishmaniasis (Leishmania-Impfstoff) bereitzustellen, mit dem ohne Nebenwirkungen ein wirksamer Schutz erzielt werden kann. Diese Aufgabe wird mit dem Gegenstand der vorliegenden Erfindung gelöst.

Gelöst wird diese Aufgabe durch einen Parasitose-Impfstoff, der dadurch gekennzeichnet ist, daß er als Wirkstoff wenigstens ein Membranantigen des Parasiten, das einen Schutz gegen Infektion mit dem homologen Parasiten hervorruft, welches in die Lipiddoppelschicht von Liposomen inkorporiert vorliegt, enthält. Zweckmäßige Ausgestaltungen davon sind Gegenstand der Ansprüche 2 bis 11.

Der erfindungsgemäße Impfstoff enthält als Wirkstoff ein oder mehrere Membranantigene des Parasiten inkorporiert in Liposomen aus natürlichen und/oder synthetischen Lipiden. Die Antigene liegen dabei mit hydrophoben Resten in der Lipiddoppelschicht des Liposoms verankert vor. Auf diese Weise ist es möglich, den Wirkstoff (die als Membran-Antigene isolierten Membran-Komponenten) in seiner nativen, nicht denaturierten Konformation anzubieten. Man kann annehmen, daß auf diese Weise, d. h. bei Verwendung der Membran-Komponenten in der Form, in der sie im Parasiten-Plasmalemma auftreten, die nachfolgende Immunantwort verbessert wird.

Der erfindungsgemäße Impfstoff kann das Membran-Antigen in Form eines Membran-Rohextraktes des Parasiten enthalten. Eine solche ungereinigte Membran-Fraktion enthält nicht nur Plasmamembranen, sondern auch intrazelluläre Membranen. Vorzugsweise enthält der erfindungsgemäße Impfstoff die wirksamen Komponenten der Membran-Fraktion aber in Form einer oder mehrerer gereinigter Membran-Antigene. Die Membran-Antigene werden unter Verwendung von Methoden gereinigt, die sicherstellen, daß sie im Impfstoff im nativen Zustand vorliegen. Insbesondere enthält der Impfstoff kein zur Behandlung von Menschen unzulässiges Adjuvans.

In der bevorzugten Form des erfindungsgemäßen Impfstoffes als Leishmania-Impfstoff, wie er in den Beispielen beschrieben wird, eignen sich besonders die Membran-Antigene der Promastigote. Der Membran-Antigen-Wirkstoff enthält dann vorzugsweise oder besteht insbesondere aus das/dem Glykoprotein gp63 oder/und das/dem Glykolipid EF ("exkretfaktor"). So zeigte z. B. die Beimpfung von Mäusen (BALB/c, CBA/Ca) mit gp63 oder EF, inkorporiert in Liposomen, eine starke Schutzwirkung gegen L. m.

mexicana, und es wurden keine verschlimmerten Reaktionen bei nachfolgender Infektion beobachtet.

Es ist bekannt, daß diese beiden als Membranantigen-Wirkstoff erfindungsgemäß bevorzugt verwendeten Antigene gp63 und EF bei der Anlagerung des Parasiten an seine Wirtszelle, den Makrophagen, beteiligt sind (vgl. z. B. E. Handman und J. Goding, EMBO. J. 4 (1985) 7, 329-336; D.G. Russell und H. Wilhelm, J. Immunol. 136 (1986) 2613-2620; E. Handman und GEF. Mitchell, Proc. Natl. Acad. Sci. USA 82 (1985) 5910-5914; G.F. Mitchell und E. Handman, Parasite Immunol. 8 (1986) 255-263). Es ist weiter bekannt, daß beide Antigene in verwandter Form bei allen Species von Leishmania, die bis heute geprüft wurden, auftreten (vgl. R.J. Etges et al, Mol. Biochem. Parasitol 14 (1985) 141-149; V. Colomer-Gould et al, J. Exp. Med. 162 (1985) 902-916; E. Handman et al, EMBO. J. 3 (1984) 2301-2306). Die Verteilung dieser Antigene während des L. m. mexicana-Lebenszyklus wurde durch Immunofluoreszenz unter Verwendung monoklonaler Antikörper verfolgt. Sowohl gp63 als auch EF sind im gesamten Promastigoten-Plasmalemma vorhanden. In den Amasti goten ist EF offensichtlich im Plasmalemma nicht vorhanden, wird aber synthetisiert und in die parasitophore Vakuole freigesetzt. Im Gegensatz dazu ist gp63 eine geringfügige Komponente der Amastigoten-Oberfläche und wird nicht in den Makrophagen freigesetzt.

Das Glykoprotein gp63 kann z. B. aus einem Detergenzextrakt einer rohen Promastigoten-Membranfraktion durch Lectin-Affinitäts-Chromatographie und AnionenaustauschChromatographie isoliert werden (vgl. z. B. D.G. Russell und H. Wilhelm, l.c.). Das Glykolipid EF kann z. B. aus der Durchflußfraktion der Lectin-Säule durch Immuno-Affinitäts-Chromatographie an WIC 108.3-Antikörper-Sepharose in gereinigter Form isoliert werden, oder auf die folgende Weise: der durch die Concanavalin-A-Sepharose-Säule laufende lösliche Extrakt wird auf eine Säule appliziert, die aus Ricinius communis-Typ-II-Sepharose besteht; das gebundene EF wird gewaschen und dann mit 0,5 M D-Galactose in Waschlösungspuffer eluiert. Die Galactose wird dann durch erschöpfende Dialyse entfernt. Die isolierten Antigene können durch SDS PAGE und Immunoblotting analysiert werden.

Überraschenderweise wurde gefunden, daß das als Wirkstoff des erfindungsgemäßen Impfstoffes in der Ausführungsform eines Leishmania-Impfstoffes verwendete System Membran-Antigen/Liposom die entsprechende Schutzwirkung bereits bei sehr geringen Antigen-Konzentrationen zeigt. Eine Erklärung für diesen unerwarteten Befund könnte sein, daß sowohl gp63 als auch EF sich an entsprechende Rezeptoren auf der Makrophagenoberfläche anlagern können. Nach Injektion können also die Membran-Antigen/Liposomen rasch und spezifisch in die Makrophagen aufgenommen werden. Die Makrophagen würden sodann die Antigene in wirksamer Weise dem Immunsystem präsentieren.

Aus diesen Ergebnissen wird aber klar, daß unabhängig vom Wirkungsmechanismus, der auf dem System Membran-Antigen/Liposom beruhende erfindungsgemäße Impfstoff zwei große Vorteile besitzt. Im Gegensatz zu vielen Adjuvantien sind Liposomen physiologisch neutral und es kann sogar via subcutaner Impfung eine Schutzwirkung erreicht werden, ohne daß irgendwelche Verschlimmerungen der Infektion auftreten.

Als Lipide für das erfindungsgemäße System Membran-antigen/Liposom können natürliche und/oder künstliche (synthetische) Lipide sowie deren Gemische verwendet werden, wobei in erster Linie solche Lipide verwendet werden, die für ähnliche Zwecke bereits bekannt sind. Solche Lipide oder deren Gemische sollen die Fähigkeit besitzen, stabile Vesikeln (Liposomen) zu bilden und die Antigene sollen nach Entfernung des Detergens in effektiver Weise und in einem im wesentlichen nativen Zustand eingebaut werden können. Bevorzugte Lipide sind z. B. eine Mischung aus Phospholipid und Cholesterin; als Phospholipid wird insbesondere ein solches verwendet, das Sojalecithin und/oder Dicetylphosphat enthält oder daraus besteht, oder ein aus Crithidia fasciculata gewonnenes Phospholipid, wie es z. B. bei D.G. Russel und H. Wilhelm, l.c. beschrieben wird.

Das Gewichtsverhältnis von Liposomenmaterial zu Membran-Antigen ist nicht kritisch; aus Praktikabilitätsgründen wird vorzugsweise ein Verhältnis Liposomenmaterial/Membranantigen von 1000/0,5 bis 50 verwendet.

Die nachfolgenden Beispiele erläutern die Erfindung anhand eines Leishmaniose-Impfstoffes näher, wobei auch auf die Figuren Bezug genommen wird, ohne die Erfindung darauf zu begrenzen. Wenn nicht anders angegeben, beziehen sich Temperaturangaben auf die Celsius-Skala, Teile-und Prozentangaben auf Gewichtsteile und Gewichtsprozent.

**Beispiel 1**

Beispiel 1A

gp63 und EF wurden aus Promastigoten des Stammes Leishmania mexicana mexicana Nr. MNYC/BZ/72/M379 * wie folgt isoliert:

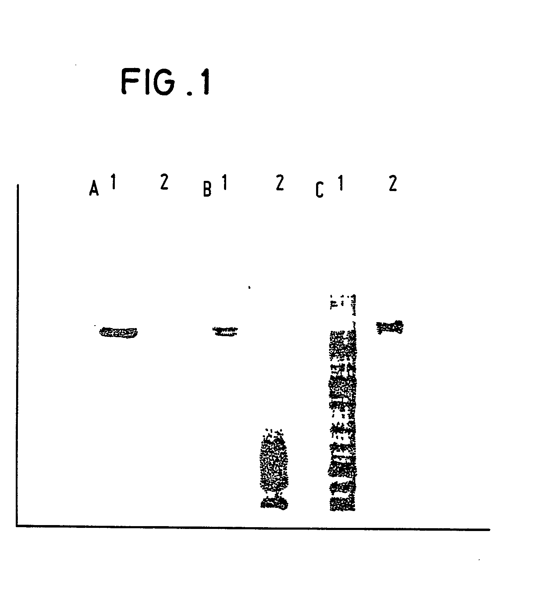

die Promastigoten wurden in SDM 79 (ein von Brun und Schönenberger in Acta Tropica (Basel) (1979) 36 289 beschriebenes Kulturmedium) kultiviert und in stationärer Phase bei einer Dichte von ca. $7,0 \times 10^7$ Zellen/ml geerntet. Die Zellen wurden in PBS (durch Phosphat bei pH = 7,2 gepufferte physiologische Kochsalzlösung) gewaschen und eine rohe Membranpräparation unter Zuhilfenahme einer Stickstoffbombe (Gerät der Firma Parr Instruments, Moline, IL, in dem Zellen durch plötzliche Druckerniedrigung zum Platzen gebracht werden) hergestellt. Die Membranen wurden dann in einer Lösung von 1 % Octylglukosid in PBS, 50 μM Leupeptin (kommerziell erhältlicher Proteaseinhibitor) und 50 μM TLCK (N-Alpha-p-tosyl-L-lysin chlormethylketon, kommerziell erhältlicher Proteaseinhibitor) suspendiert und gelöst. Der lösliche Extrakt wurde über eine Concanavalin-A-Sepharose-Säule (Firma Pharmazia) geführt. Das EF lief durch die Säule, während das gp63 gebunden wurde. Das gp63 wurde weiter durch Anionenaustausch-Chromatographie an DEAE-Sephadex oder mit Hilfe eines anti gp63 monoklonalen Antikörpers bis zu der in Fig. 1, A1, B1, C2 gezeigten Homogenität gereinigt. EF wurde durch Inkubation mit WICI 08.3-Sepharose isoliert und das gebundene Material mit 6 M Guanidiniumhydrochlorid eluiert. Das Guanidiniumhydrochlorid wurde durch erschöpfende Dialyse vor der Verwendung entfernt (zur Durchführung des Verfahrens vgl. auch D.G. Russell und H. Wilhelm, l.c.).

Das SDS-Polyacrylamidgel in Fig. 1 vermittelt einen Eindruck von der Reinheit der zur Immunisierung verwendeten Antigene. Für A und B wurden die Promastigoten mit ($^3$H)-Galactose bzw. ($^{35}$S)-Methionin markiert. A1, gereinigtes gp63 nach Anfärbung mit Coomassie-Blau; A2, gereinigtes EF, nicht mit Coomassie-Blau anfärbbar; B1, B2, Autoradiographie des gleichen Gels, ($^3$H)-Galactose wird sowohl in gp63 (B1), als auch EF (B2 verschmierte Bande vom Molekulargewicht 10 bis 35.000) eingebaut. C1, Autoradiogramm eines Lysats von ($^{35}$S)-Methionin markierten Promastigoten; C2, aus diesem Lysat durch den monoklonalen Antikörper TÜL 3.8 immunpräzipitiertes gp63.

Beispiel 1B:

Die die Promastigoten-Antigene enthaltenden Liposomen wurden durch Detergenzien-Dialyse, wie bei G.D. Russell und H. Wilhelm, l.c., beschrieben, hergestellt, aber mit den folgenden Abänderungen: die Phospholipide wurden aus den trypanosomatiden Geiseltierchen Crithidia fasciculata, mit Chloroform/Methanol extrahiert, über eine Kieselgelsäule von Verunreinigungen befreit, getrocknet und in Octylglucosid oder Zwittergent 3.12 und Chloroform gelöst. Cholesterin wurde zu der Mischung bis zu 15 % der Phospholipidkonzentration zugegeben. Die Mischung wurde dann zu einem dünnen Film unter Stickstoff getrocknet und eine Stunde lang bei 37°C belassen. Die Parasiten-Antigene (in PBS und Detergens) wurden dann zu dem Lipidfilm hinzugefügt und einer Beschallung in einem Eisbad unterworfen. Bei allen Versuchen wurde ein Verhältnis von 2 μg Parasit-Antigen zu 1 mg Phospholipid/ Cholesterin aufrechterhalten. Das Detergens wurde durch Dialyse (über Nacht) gegen PBS bei 4°C entfernt. Die erhaltenen Vesikel wurden durch Zentrifugation bei 100.000 g während einer Stunde bei 4°C erhalten und mehrmals in PBS gewaschen. Die Liposomen wurden für die Immunisierungsversuche sofort verwendet, obgleich eine Lagerung bei -20°C keine Änderung ihrer Stabilität ergab.

Beispiel 2

Die Tabelle I zeigt die verschiedenen verwendeten Liposomen-Präparationen und die in der Maus kurz vor der Exposition mit homologem Leishmania gemessenen relativen Antikörper-Titer.

Tabelle I

* DSM 4308; aus Sammlung der London School of Hygiene and Tropical Medicine

Beimpfungsgruppe und relative Anti-Leishmania-Antikörper-Titer. (Der Antikörper-Titer wird ausgedrückt als die Menge von $^{125}$-I-markiertem Protein A, in cpm (Impulse pro Minute; Kontrolle = 0), das an die Leishmania-Antigen-Platten nach Inkubation im Antiserum gebunden ist).

|  | | BALB/c | CBA/Ca |
|---|---|---|---|
| 1. Kontrolle | | 0 | 0 |
| 2. Liposomen | (s.c.) | ND | ND |
| | (i.p.) | ND | ND |
| 3. Rohe Antigen-Liposomen | (s.c.) | 151 | 51 |
| | (i.p.) | 303 | 420 |
| 4. gp63 Liposomen | (s.c.) | 32 | 42 |
| | (i.p.) | 186 | 408 |
| 5. EF Liposomen | (s.c.) | 3 | 12 |
| | (i.p.) | 38 | -15 |
| 6. gp63/EF Liposomen | (s.c.) | 62 | 21 |
| | (i.p.) | 385 | 347 |
| 7. gp63/EF Liposomen + CFA | (s.c.) | 123 | 12 |
| | (i.p.) | 1080 | 132 |
| 8. gp63/EF Liposomen | (i.v.) | 236 | 348 |

ND = nicht durchgeführt
CFA = vollständiges Freund'sches Adjuvans

Alle Mäuse wurden wie folgt behandelt:
Die Gruppen umfaßten jeweils 6 10 Wochen alte weibliche Mäuse, BALB/c oder CBA/Ca, erhalten von Olac-Limited, Großbritannien. Die anfängliche Immunisierung bestand aus 5 μg gp63 oder 4 μg EF-Antigen, eingebaut in 2,5 mg C. fasciculata-Phospholipid, mit 15 % Cholesterin, in 50 μl PBS, die jeder Maus entweder subcutan oder intraperitoneal verabreicht wurden. Die Kontrollgruppen bestanden aus Mäusen, die entweder mit Liposomen ohne Parasit-Antigen oder einem vergleichbaren Volumen PBS beimpft wurden. Vier Wochen später wurden die Mäuse mit einer ähnlichen, wiederholten Dosis gespritzt und dann vier Wochen aufbewahrt, bevor ihnen eine subcutane Infektion mit $5 \times 10^4$ L. m. mexicana-Promastigoten (stationäre Phase) an einer geschorenen Stelle am Rücken verabreicht wurde. Jeder Maus wurde zur Zeit der Infektion eine Serumprobe entnommen, um den relativen Titer von Anti-Leishmania-Antikörpern zu bestimmen.

Gleiche Ergebnisse wurden mit einer Liposomen-Zusammensetzung von 80 % Sojalecithin, 15 % Cholesterin und 5 % Dicetylphosphat erhalten.

Der Antikörper-Titer wurde durch Radioimmuno-Assay wie folgt bestimmt: L. m. mexicana-Membranen (D.G. Russell und H. Wilhelm, l.c.) in 1 % Nonidet P 40 in PBS, mit Proteaseinhibitor, wurden extrahiert. Nach Zentrifugation bei 100.000 g während 30 Minuten bei 4°C wurde der lösliche Extrakt mit Wasser verdünnt und auf Polyvinyl-Mikrotiterplatten getrocknet. Die Platten wurden durch Inkubation während 60 Minuten mit 10 MM Tris (pH = 7,4), 140 mM NaCl und 0,5 % Tween 20 gegen eine nicht spezifische Proteinabsorption mit 10 mg/ml BSA blockiert. Die Antiseren wurden in dem obigen Puffer 50:1 verdünnt, in Duplikaten in die Vertiefungen der Mikrotiterplatte gegeben und 90 Minuten bei 37°C inkubiert. Die Platten wurden gewaschen und 30 Minuten bei 20°C mit $^{125}$J-markiertem Protein A im gleichen Puffer inkubiert. Schließlich wurden die Platten gewaschen, bis in dem Waschpuffer keine freie Radioaktivität mehr bestimmbar war, getrocknet und in einem Beckmann-Gamma-Zähler ausgezählt. Die Werte der Anti-Leishmania-Anti-Seren werden als mittlere cpm (Impulse pro Minute) jeder Gruppe gegenüber dem Kontrollwert (nicht immunisiertes Mäuseserum, Kontrollwert = 0) ausgedrückt.

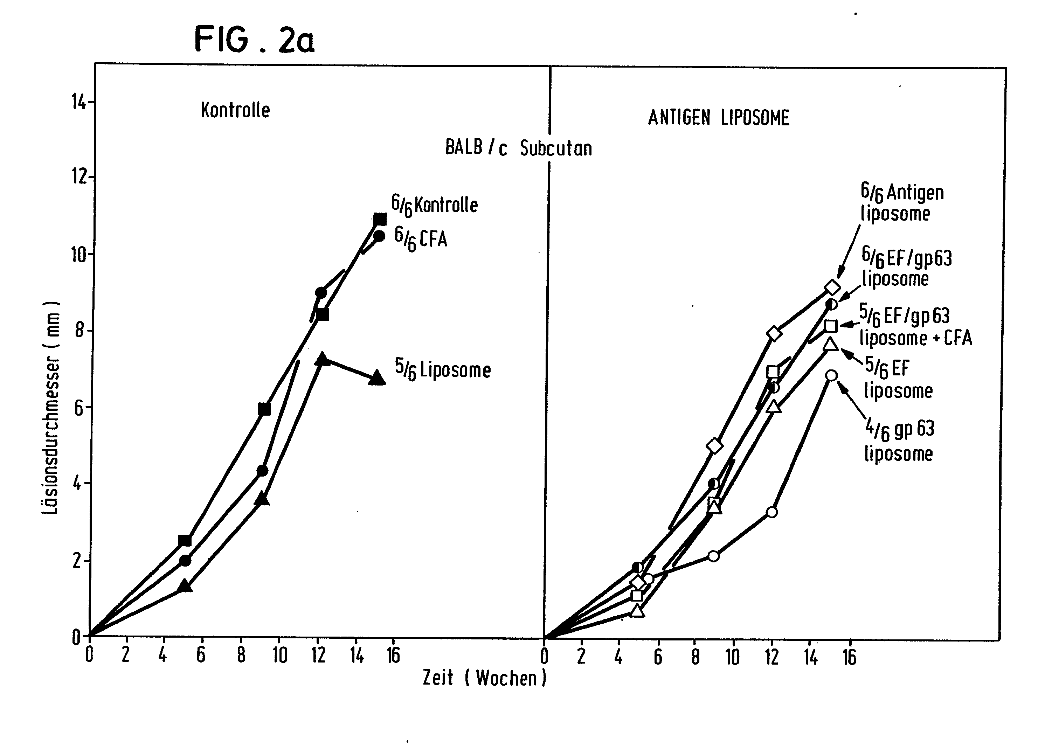

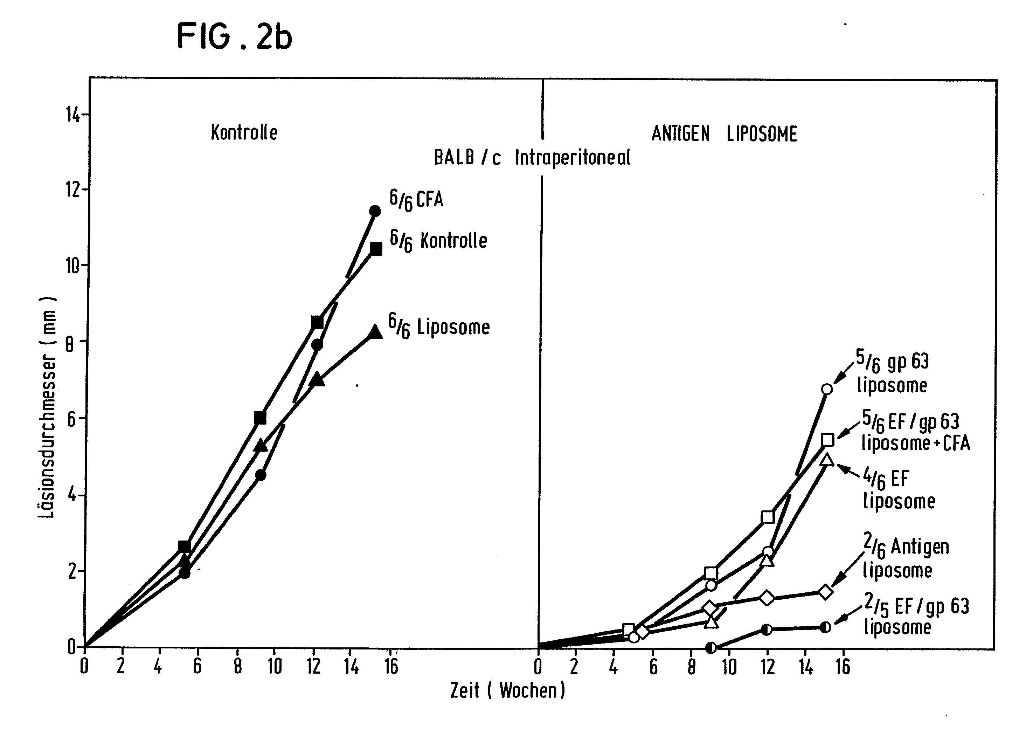

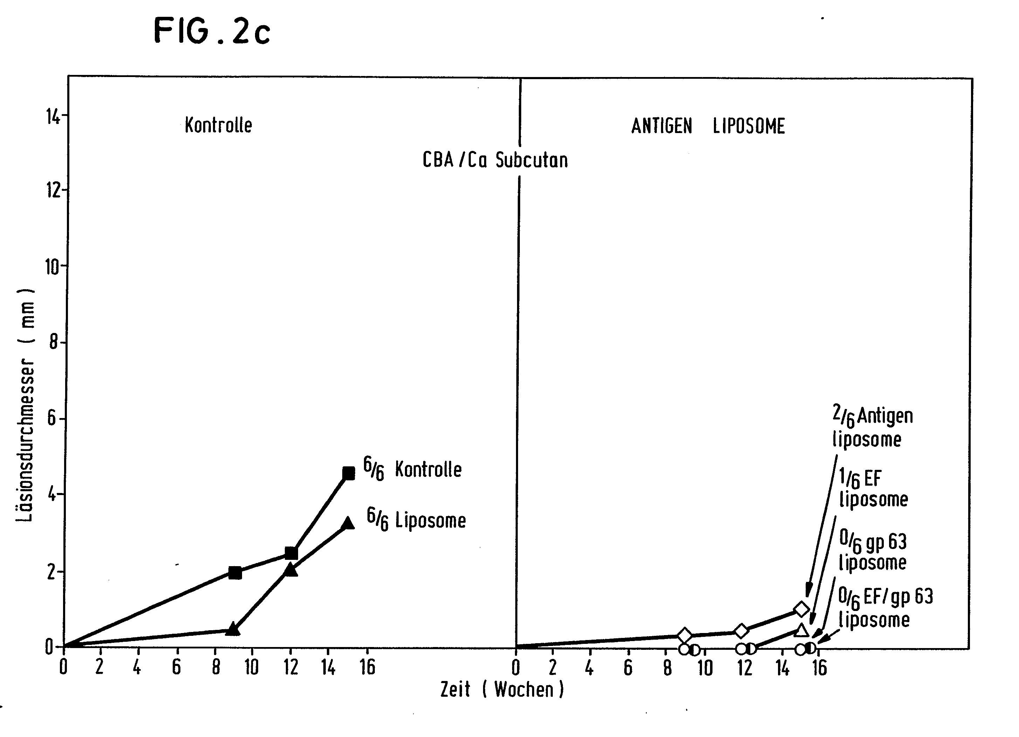

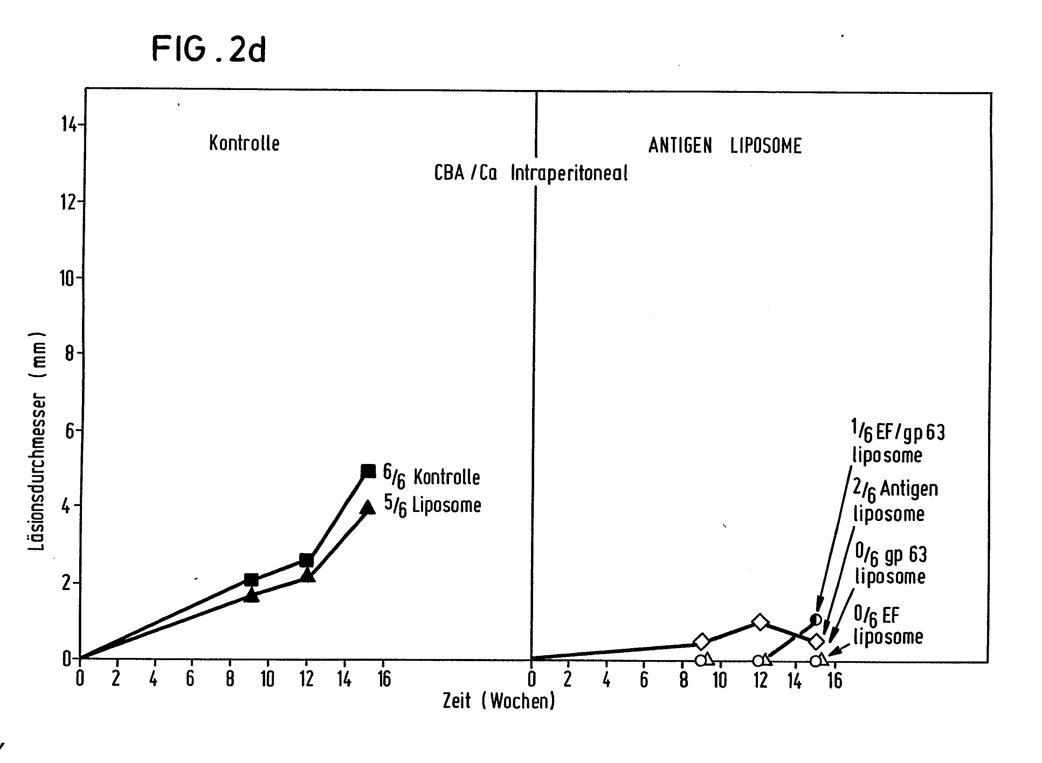

Die Figur 2 zeigt graphisch die Entwicklung von L. m. mexicana-Läsionen in BALB/c (Fig. 2a und 2b) und 3CBA/Ca (Fig. 2c und 2d) Mäusen nach Immunisierung mit verschiedene Antigene enthaltenden Liposomen und Exposition mit homologen Parasiten. Die Art der Beimpfung umfaßte subcutane (Fig. 2a und 2c) und intraperitoneale (Fig. 2b und 2d) Injektionen mit 3eder der verschiedenen Antigen-Präparation. Der Durchmesser der Läsionen wurde in zwei Richtungen gemessen und als mittlerer Läsionsdurchmesser aller Mäuse in einer Gruppe ausgedrückt. Die Zunahme in der Größe der Läsion wird gegen die Zeit (in Wochen ab Exposition) aufgetragen. Das neben dem Endwert jeder Gruppe angegebene Verhältnis bezeichnet die Zahl infizierter Individuen gegenüber der Gruppengröße.

### Ansprüche

1. Parasitose-Impfstoff, **dadurch gekennzeichnet,** daß er als Wirkstoff wenigstens ein Membranantigen des Parasiten, das einen Schutz gegen Infektion mit dem homologen Parasiten hervorruft, in die Lipiddoppelschicht von Liposomen inkorporiert enthält.

2. Impfstoff nach Anspruch 1, **dadurch gekennzeichnet,** daß er einen Membran-Rohextrakt oder ein oder mehrere gereinigte Membranantigene enthält.

3. Impfstoff nach Anspruch 1 oder 2, **dadurch gekennzeichnet,** daß das Membranantigen in nativem Zustand vorliegt.

4. Impfstoff nach einem der vorhergehenden Ansprüche, **dadurch gekennzeichnet,** daß die Liposomen aus einer Mischung von Phospholipid und Cholesterin bestehen.

5. Impfstoff nach Anspruch 4, **dadurch gekennzeichnet,** daß das Phospholipid Sojalecithin und Dicetylphosphat enthält oder daraus besteht. ·

6. Impfstoff nach Anspruch 5, **dadurch gekennzeichnet,** daß das Phospholipid aus Crithidia fasciculata gewonnen ist.

7. Impfstoff nach einem der vorhergehenden Ansprüche, **dadurch gekennzeichnet,** daß das Gewichtsverhältnis von Liposomenmaterial zu Membranantigen 1000:0,5 bis 50 beträgt.

8. Impfstoff nach einem der vorhergehenden Ansprüche, **dadurch gekennzeichnet,** daß er frei von Adjuvans ist.

9. Impfstoff nach einem der vorhergehenden Ansprüche, **dadurch gekennzeichnet,** daß er ein Membranantigen von Leishmania enthält.

10. Impfstoff nach Anspruch 9, **dadurch gekennzeichnet,** daß das Leishmania-Membranantigen von der Promastigote stammt.

11. Impfstoff nach Anspruch 9 oder 10, **dadurch gekennzeichnet,** daß er als Membranantigen das Glykoprotein gp63 oder/und das Glykolipid EF ("Exkretfaktor") enthält oder daraus besteht.

12. Verfahren zur Herstellung des Impfstoffs nach einem der Ansprüche 1 bis 11, **dadurch gekennzeichnet,** daß man das Liposomenmaterial und Octylglucosid in einem polarem organischen Lösungsmittel löst, zu einem Film trocknet, das Parasitenmembranantigens in Octylglucosid enthaltender wäßriger Lösung zu dem Film gibt und mit Ultraschall behandelt und danach das Octylglucosid aus der Mischung durch Dialyse entfernt.

6

# FIG .1

FIG . 2a

# FIG. 2b

Kontrolle

ANTIGEN LIPOSOME

BALB / c Intraperitoneal

6/6 CFA

6/6 Kontrolle

6/6 Liposome

5/6 gp 63 liposome

5/6 EF / gp 63 liposome + CFA

4/6 EF liposome

2/6 Antigen liposome

2/5 EF / gp 63 liposome

Läsionsdurchmesser ( mm )

Zeit ( Wochen )

0 276 783

# FIG.2c

Kontrolle

CBA /Ca Subcutan

ANTIGEN   LIPOSOME

Läsionsdurchmesser ( mm )

Zeit ( Wochen )

6/6 Kontrolle

6/6 Liposome

2/6 Antigen liposome

1/6 EF liposome

0/6 gp 63 liposome

0/6 EF/ gp 63 liposome

0 276 783

**FIG.2d**

Kontrolle

CBA/Ca Intraperitoneal

ANTIGEN LIPOSOME

Läsionsdurchmesser (mm)

Zeit (Wochen)

6/6 Kontrolle

5/6 Liposome

1/6 EF/gp63 liposome

2/6 Antigen liposome

0/6 gp 63 liposome

0/6 EF liposome

0 276 783